# FASCICULE DE BREVET EUROPEEN

(11) **EP 0 894 785 B1**
(45) Date de publication et mention de la délivrance du brevet: **14.11.2001**
(21) Numéro de dépôt: 98401814.3
(22) Date de dépôt: 17.07.1998
(51) Int. Cl.: C07C 67/347, C07C 69/587

(54) **Procédé pour la codimérisation de corps gras polyinsaturés et d'oléfines**
Verfahren zur Codimerisation von polyungesättigten Fettverbindungen und Olefinen
Process for codimerizing polyunsaturated fatty compounds and olefines

(30) Priorité: 25.07.1997 FR 9709615
(43) Date de publication de la demande: 03.02.1999
(73) Titulaire: INSTITUT FRANCAIS DU PETROLE, 92500 Rueil Malmaison (FR)
(72) Inventeur: Hillion, Gérard, 95220 Herblay (FR); Olivier, Hélène, 92500 Rueil Malmaison (FR); Siepen, Katja, 40699 Erkrath (DE); Stern, Robert, 75017 Paris (FR)
(74) Mandataire: Andréeff, François

(56) Documents cités:
- EP-A- 0 621 250
- EP-A- 0 621 257

## Description

L'invention a pour objet un nouveau procédé d'obtention de composés chimiques issus de corps gras polyinsaturés, composés caractérisés par la présence le long de la chaîne linéaire hydrocarbonée d'une ou de plusieurs ramifications de deux atomes de carbone au moins.

Ces composés sont obtenus par addition d'oléfines sur des corps gras polyinsaturés en présence d'un système catalytique au cobalt.

Ces co-dimères insaturés peuvent être hydrogénés et l'on obtient alors des corps gras saturés caractérisés par un point de fusion généralement inférieur à -20°C, une thermostabilité importante et des propriétés tensio-actives recherchées. Ces propriétés sont particulièrement intéressantes dans les applications suivantes : lubrifiants, émulsifiants, fluides de forage, bactéricides, solvants et sous forme de sels pour dissoudre des métaux lourds dans des solvants apolaires.

La réaction des oléfines avec le butadiène ou d'autres diènes est connue depuis longtemps et a fait l'objet de plusieurs revues. La codimérisation du butadiène avec l'éthylène conduit à l'hexadiène-1,4, celle de l'éthylène avec l'isoprène au méthyl-3 hexadiène et enfin, par codimérisation de l'éthylène avec le pipérylène, on obtient le vinyl-2-pentène. De nombreux catalyseurs sont utilisés pour réaliser ces réactions. On peut citer par exemple les systèmes au rhodium, au ruthénium, au palladium, au cobalt, au fer ou au nickel. Les systèmes à base de titane ont été décrits (Connel, Laurence G - Ann. N.Y. Acad Sci (73), 214, 143-9) pour catalyser la formation du vinylcyclobutane à partir d'éthylène et de butadiène.

Par contre, l'addition d'oléfine sur des diènes fonctionnels n'a été que très peu décrite. Le brevet US-A-3742080 signale la possibilité d'additionner de l'éthylène à des diènes dont l'une ou les deux extrémités des chaînes hydrocarbonées sont substituées par des groupes halogènes ou alkoxy.

Il est également connu qu'une oléfine peut réagir sur un composé diénique ou triénique conjugué selon une réaction de type Diels-Alder. Il est décrit (R.E. Beal et Coll. JAOCS 52, 400 (1975)) par exemple l'addition d'éthylène sur des corps gras polyinsaturés par simple chauffage à une température de 290 °C. Ainsi, à partir de linoléate de méthyle et d'éthylène, on obtient un composé possédant dans sa chaîne hydrocarbonée un cycle insaturé à 6 atomes de carbone. Après hydrogénation, ces composés possèdent des propriétés intéressantes. Toutefois, leur point de fusion, supérieur à 10 °C, est encore trop élevé pour permettre leur utilisation comme lubrifiants.

On connait une autre méthode d'obtention de composés ramifiés de corps gras. Elle consiste à faire réagir, selon une réaction de type Wittig, une cétone comme par exemple l'ester méthylique de l'acide 12- oxo octadécanoïque avec un ylure, par exemple l'intermédiaire P(⌀)₃=CHCH₃, où Ø représente un radical phényle. On obtient alors le composé CH₃(CH₂)₅C(=CHCH₃)(CH₂)₁₀COOCH₃, qui peut être hydrogéné en éthyl-12-octadécanoate de méthyle. (D.G. Chasin et Coll, Chem. Phys. Lipids (71) 6, 8-30).

On a signalé également dans la nature la présence de composés saturés ramifiés de corps gras tirés des bacilles de Koch par exemple, ou, avec une autre longueur de chaîne hydrocarbonée, dans le suif de mouton.

On sait enfin que les produits que l'on appelle «isostéariques» contiennent des traces de composés portant des ramifications de type éthyle ou vinyle.

Récemment, les demandes de brevets internationales WO-A-91/11428, 91/11427, 91/11426 et 91/11425 décrivent l'obtention de composés de corps gras ramifiés par un procédé catalytique. L'addition de l'oléfine, telle que l'éthylène ou le propylène, sur le corps gras polyinsaturé, un ester de l'acide linoléique par exemple, est catalysée par un système à base de rhodium, d'iridium, de palladium ou de ruthénium. Les systèmes au rhodium, qui sont les seuls à avoir été décrits de façon évidente, restent cependant peu actifs.

Les brevets US-A-5 476 956 et 5 434 282 correspondant à EP-A-O 621 250 et 0 621 257, décrivent l'utilisation d'un système catalytique au rhodium très spécifique, qui permet d'accélérer l'addition de l'oléfine sur les diènes de corps gras, notamment les diènes conjugués, d'un facteur 50 à 100. Toutefois, ce procédé reste encore difficilement applicable à une grande échelle en raison de la consommation trop élévée de rhodium.

L'objet de la présente invention est un nouveau procédé de codimérisation d'un composé monooléfinique avec un corps gras polyinsaturé en présence d'un système catalytique comprenant un composé de cobalt. Grâce à ce type de catalyseur, on peut envisager de réduire fortement le coût de cette réaction et aussi obtenir une sélectivité améliorée par rapport aux produits obtenus avec les complexes au rhodium.

Le composé dit «corps gras polyinsaturé» mis en jeu dans la réaction sur laquelle est basée le procédé de l'invention est en général un composé comprenant d'une part au moins deux liaisons éthyléniques, ces liaisons pouvant être conjuguées ou conjugables deux à deux et d'autre part une fonction carboxylique comme celle présente dans les acides gras ayant de 18 à 26 atomes de carbone. Les huiles de tournesol, de carthame, de poisson, de lin, de soja, d'oïticica, de coton, de colza, de bois de chine, de noix, de maïs, de linola, de pépins de raisin et généralement toutes les huiles ou leurs esters dérivés comprenant des composés polyinsaturés sont des matières premières envisageables.

Les acides gras considérés, diéniques, triéniques ou polyéniques peuvent être utilisés tels quels ou, de préférence, sous la forme de leurs esters, que l'on forme à partir d'acides gras par réaction avec des alcools monofonctionnels, tels que le méthanol ou l'éthanol, difonctionnels, tels que le néopentylglycol, trifonctionnels, tels que le triméthylolpropane, polyfonctionnels tels que le sorbitol, les polyglycérols, le pentaérythritol et les sucres. Les huiles elles-mêmes sont des substrats possibles.

Ces esters peuvent être conjugués partiellement ou totalement. Autrement dit, ils peuvent contenir au moins deux doubles liaisons éthyléniques séparées ou non entre elles par un groupe méthylénique. Parmi les procédés de conjugaison des doubles liaisons les plus connus, on peut citer ceux qui utilisent les alcoolates alcalins en présence ou non d'un solvant. On peut dans ce cas obtenir jusqu'à 99 % de corps gras conjugué par rapport au corps gras polyinsaturé présent initialement dans l'huile.

On connait d'autres systèmes catalytiques conjugants, mettant en jeu des complexes de ruthénium ou le fer carbonyle. Le système au cobalt est lui-même conjugant. On peut lui adjoindre pour la conjugaison un co-métal.

Le composé monooléfinique mis en jeu dans la réaction peut consister en toute oléfine réactive choisie parmi les monooléfines ordinaires (hydrocarbures monoléfiniques), telles que par exemple l'éthylène, le propylène ou le butène-1.

Le procédé de préparation de corps gras ramifiés selon l'invention est caractérisé en ce que la réaction est catalysée par une composition catalytique comprenant au moins un composé de cobalt, au moins un composé réducteur et au moins un ligand contenant du phosphore, de l'arsenic, de l'antimoine ou de l'azote.

Le composé du cobalt peut être un sel inorganique ou organique bivalent ou trivalent et exceptionnellement monovalent de cobalt; on peut citer par exemple les halogénures, les thiocyanates, les sulfates, les nitrates, les alcoolates, les carbonates, les carboxylates, les bétadicétonates ou les esters d'acides bétacétocarboxyliques ; des exemples particuliers des sels de cobalt utilisables sont le chlorure de cobalt (II), le chlorure de cobalt (III), le bisacétylacétonatocobalt (II), le trisacétylacétonatocobalt (III) et les acétates de cobalt (II) et (III); on peut également utiliser les hydroxydes de cobalt, les composés organo-cobalt et les hydrures de cobalt.

Comme composés réducteurs, on peut citer les organoaluminiques de formule générale RₓAlX₃₋ₓ ou R₄Al₂SO₄, où R est l'hydrogène ou un groupe alkyle, où X est un halogène, et x = 1, 2 ou 3, les organomagnésiens de formule RMgX, avec R = alkyle et X = halogène, les aluminoxanes, le borohydrure de sodium NaBH₄ et des hydrures alcalins variés, tels que LiAlH₄ et NaAlH₄, eux-mêmes ou leurs dérivés obtenus en substituant de 1 à 3 atomes d'hydrogène par 1, 2 ou 3 groupements alcoxy, par exemple LiAlH₃(OR), LiAlH₂(OR)₂ et LiAlH(OR)₃, où R = groupement alkyle, par exemple méthyle, éthyle, isopropyle, butyle, isobutyle ou terbutyle, etc. ;

Le ligand peut être choisi :
- parmi les composés du phosphore de formule PRₘX_{3-m,} avec m = 0, 1, 2 ou 3, R = aryle ou alkyle et X = halogène ; les phosphites P(OR)₃, avec R = aryle ou alkyle ; les oxydes de phosphine POR₃ et les diphosphines de formule R₂P-(CH₂)ₙ-PR₂, avec R = aryle ou alkyle et n = 0 - 4 ;
- parmi les composés analogues de l'arsenic et de l'antimoine ;
- et parmi les ligands azotés, tels que les dérivés amidiques, les imines ou diimines (fabriqués par exemple par réaction du glyoxal avec un dérivé de l'aniline substitué sur le noyau aromatique) et les dérivés pyridiniques, par exemple le dipyridyle.

On peut éventuellement mettre en jeu un composé organique qui joue le rôle de solvant ; comme solvants, on peut utiliser les hydrocarbures aliphatiques ou aromatiques, les éthers, les esters, les hydrocarbures halogénés et, à faible concentration, des sulfoxydes et des amides ; la réaction peut aussi être réalisée en l'absence de solvant ajouté ; c'est alors l'ester dont une partie ne réagit pas avec l'oléfine qui joue le rôle de solvant ;

On peut encore adjoindre au catalyseur de codimérisation un sel d'un autre métal de transition (par exemple Fe, Ni, Cu, Rh, Pd, Mn, Mo, W ou V, de préférence Fe, Ni, Cu, Rh ou Pd), introduit en proportion mineure par rapport au cobalt et qui permet d'accélérer la réaction, en particulier lorsque le substrat polyinsaturé à base de corps gras n'a pas ses doubles liaisons sous la forme conjuguée.

Le rapport molaire entre le ligand et le composé du cobalt est compris de préférence entre 0,5 et 30, en particulier entre 1 et 3.

Si le ligand coordonne pour un site de coordination, on a intérêt à l'utiliser avec un rapport molaire ligand/cobalt égal à 2 à 3. Si le ligand est bicoordinant on l'utilise avec un rapport molaire 1 à 1,5.

Le rapport molaire entre le réducteur et le composé du cobalt est généralement compris entre 1 et 30, de préférence entre 2 et 4.

Selon la présente invention il est possible de préformer le système catalytique en faisant réagir le sel de cobalt, le ligand et le réducteur, puis de l'introduire dans le corps gras insaturé en présence de l'oléfine.

Généralement, il est préférable d'ajouter le ligand au composé du cobalt en présence de corps gras insaturé, avant d'ajouter le réducteur. On peut aussi isoler un complexe réduit du cobalt de type CoHXL₂, avec X = halogène et L₂ une diphosphine ou deux phosphines et ajouter un alkylaluminium ou tout autre réducteur en présence de corps gras insaturé.

La composition catalytique est ajoutée au système en quantité catalytique. Cette quantité s'exprime comme étant de 10⁻⁴ à 10⁻¹ mole de cobalt par mole de corps gras insaturé conjugué. La température de réaction est comprise de préférence entre 50 et 120 °C. La pression d'éthylène ou de propylène est comprise entre 0,1 et 30 MPa de préférence entre 0,1 et 5 MPa. Les temps dépendent de la concentration et de la nature du catalyseur. Les temps peuvent être courts, par exemple de quelques minutes à quelques heures.

On peut opérer selon un procédé continu ou discontinu. L'introduction du catalyseur et des esters dans le réacteur peut se réaliser en présence d'éthylène à basse température ou à plus haute température directement dans le réacteur.

On peut opérer avec un catalyseur insoluble supporté, en fixant le cobalt sur un support phosphinique hétérogène polymérique.

Les corps gras ramifiés obtenus peuvent être hydrogénés pour obtenir des produits plus stables. L'hydrogénation des composés oléfiniques est connue depuis près de 30 ans. Elle a été réalisée notamment en 1971 par Chasin, cité plus haut. Les catalyseurs utilisables sont ceux qui sont connus pour hydrogéner des oléfines, soit le nickel de Raney, le palladium sur charbon, le nickel supporté, généralement après avoir éliminé le catalyseur de codimérisation par lavage à l'eau. On peut parfois utiliser le catalyseur de codimérisation comme catalyseur d'hydrogénation. Après hydrogénation, on élimine les composés saturés non ramifiés par cristallisation ou par distillation. On peut aussi distiller avant hydrogénation pour concentrer les produits ramifiés.

Les esters ramifiés peuvent servir de bases de lubrifiants ou d'émulsifiants, ou subir d'autres traitements comme la transestérification avec des alcools plus lourds lorsqu'on a affaire, au départ, à des esters méthyliques.

Les exemples suivants illustrent l'invention.

### EXEMPLE 1 :

### Préparation du précurseur du catalyseur

Dans un tube de Schlenk, sous atmosphère d'argon, on introduit 1,4 mmole de bisacétylacétonatocobalt (II) Co(acac)₂, 1,4 mmole de diphénylphosphinoéthane (dppe), 55 ml de toluène et 30 ml d'ester méthylique conjugué de l'huile de tournesol dont la composition est la suivante : 4,75% de C16:0, 4,41 % de C18:0, 28,5% de C18:1, 3,65 % de C18:2 non conjugué et 61,6 % de C18:2 conjugué (55 mmole). Cette suspension est chauffée à 80 °C pendant 30 minutes, ce qui conduit à une solution à laquelle on rajoute 14 mmoles de diéthylchloroaluminium (DEAC), dilué à 25 % dans le toluène.

### Catalyse de codimérisation

Dans un autoclave de 250 ml en Hastelloy®, muni d'une agitation par barreau magnétique et d'une double enveloppe et préalablement chauffé à 80 °C, on introduit sous atmosphère d'argon, la totalité de la solution précédente. Le réacteur est alors mis sous une pression de 3 MPa d'éthylène maintenue constante au cours de la réaction. Au bout de 3 heures, l'agitation est arrêtée, le réacteur est dépressurisé et ouvert. Après élimination du catalyseur par lavage à l'eau, le mélange obtenu est analysé par chrommatographie en phase vapeur sur colonne capillaire très polaire du type BP x 70 d'un diamètre de 0,32 cm et d'une longueur de 50m.

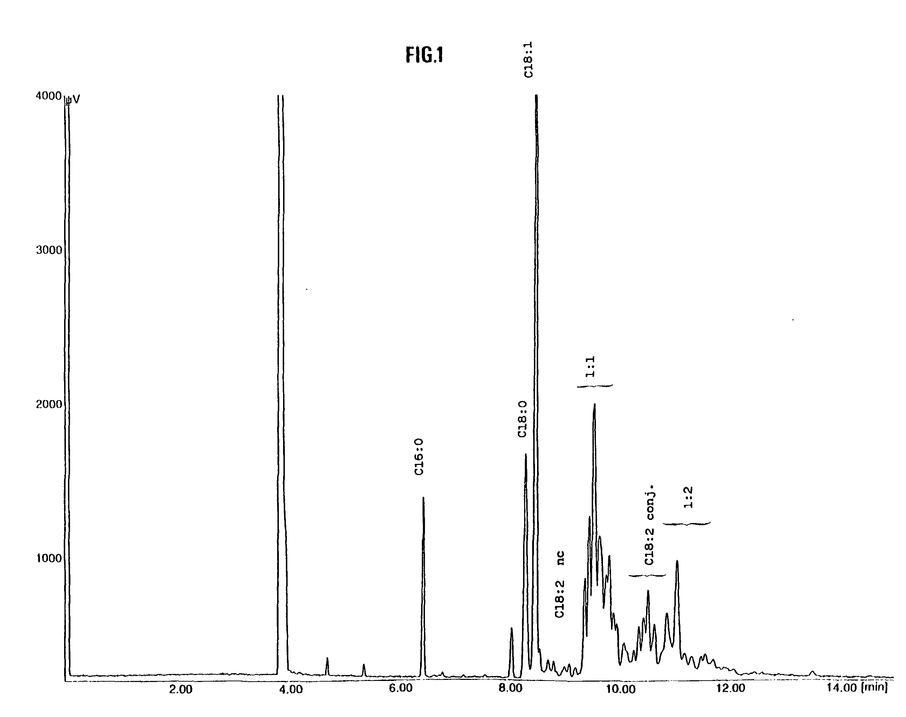

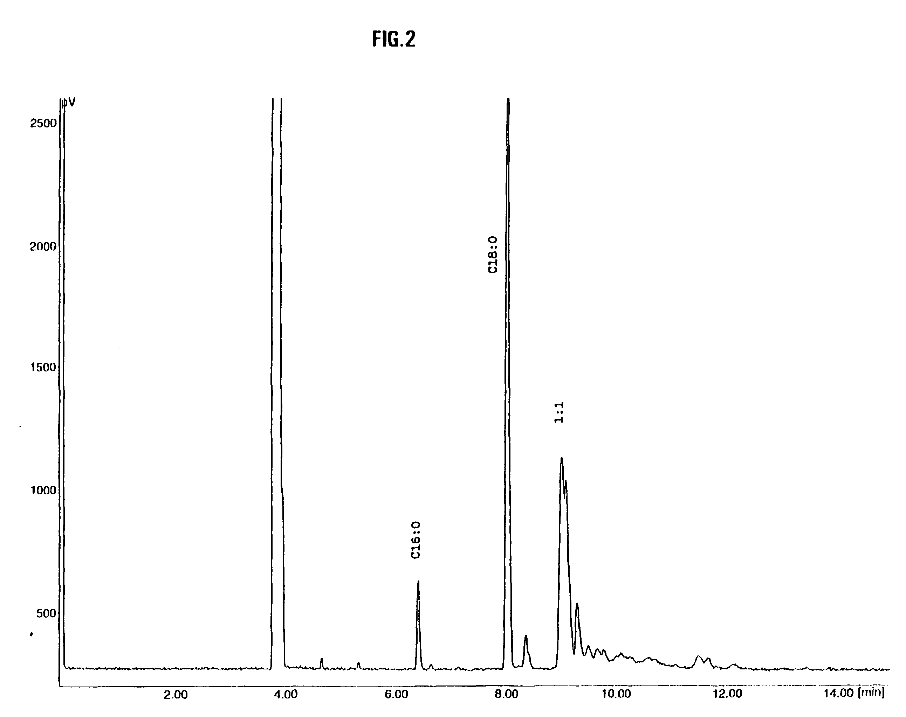

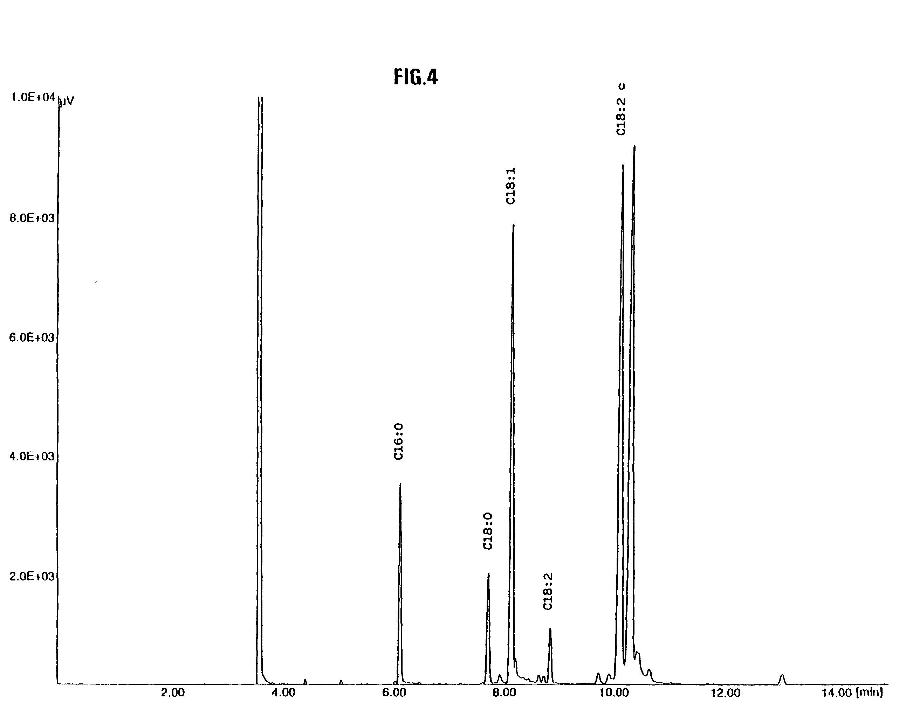

Un exemple de chromatogramme des produits de la réaction est donné en annexe (Fig.1), ainsi qu'un exemple de chromatogramme d'un produit hydrogéné (Fig.2) et du produit de départ (Fig.4).

### EXEMPLE 2 :

Dans un autoclave de 250 ml en Hastelloy®, préalablement chauffé à 80 °C, on introduit la solution préparée dans l'exemple 1 mais sans le DEAC. Celui-ci (14 mmoles dilué à 25 % dans le toluène) est introduit dans le réacteur à 80 °C sous atmosphère d'argon. Le réacteur est alors mis sous une pression constante de 3 MPa d'éthylène. La réaction est arrêtée au bout de 6 heures. Les résultats figurent dans le Tableau 2. Selon ce tableau, il n'y a pas de différence dans ces deux manières de réaliser la réaction.

### EXEMPLE 3 :

On opère comme dans l'exemple 2 mais le dichloro-1,2 éthane (30 ml) est utilisé comme solvant à la place du toluène. La réaction est arrêtée au bout de 3 heures. Les résultats figurent dans le Tableau 2.

### EXEMPLE 4 :

On opère comme dans l'exemple 3 mais sans solvant. L'ester méthylique de tournesol, qui joue le rôle de solvant, a la composition suivante : 6,30 % de C16:0, 4,72 % de C18:0, 19,2 % de C18:1, 5,29 % de C18:2 non conjugué et 63,6 % de C18:2 conjugué. La réaction est arrêtée au bout de 3 heures. Les résultats figurent dans le Tableau 2.

### EXEMPLE 5 :

On opère comme dans l'exemple 2 mais la réaction catalytique est réalisée à 100 °C. La réaction est arrêtée au bout de 3 heures. Les résultats figurent dans le Tableau 2.

### EXEMPLE 6 :

On opère comme dans l'exemple 3 mais le triéthylaluminium (TEA) est utilisé à la place du DEAC et est injecté non dilué dans le réacteur. L'ester méthylique de tournesol utilisé a la composition de celui décrit dans l'exemple 4. La réaction est arrêtée au bout de 3 heures. Les résultats figurent dans le Tableau 2.

### EXEMPLE 7 :

On opère comme dans l'exemple 2 mais avec 1,3 mmole de dppe par rapport au cobalt et le rapport molaire Co/AlEt₂Cl = 1:10. La réaction est arrêtée au bout de 3 heures. Les résultats figurent dans le Tableau 2. On remarque dans cet essai qu'il ne se forme pas de cobalt métallique.

### EXEMPLE 8 :

On opère comme dans l'exemple 6 mais le toluène est utilisé comme solvant à la place du dichloro-1,2 éthane. La réaction est arrêtée au bout de 3 heures. Les résultats figurent dans le Tableau 2.

### EXEMPLE 9 :

On opère comme dans l'exemple 2 mais on utilise le diphénylphosphinopropane comme ligand à la place du dppe. La réaction est arrêtée au bout de 4 heures. Les résultats figurent dans le Tableau 2.

### EXEMPLE 10 :

On opère comme dans l'exemple 2 mais on utilise le diphénylphosphinométhane (dppm) comme ligand. La réaction est arrêtée au bout de 4 heures. Les résultats figurent dans le Tableau 2.

### EXEMPLE 11 :

On opère comme dans l'exemple 2 mais on utilise la triphénylphosphite comme ligand. La réaction est arrêtée au bout de 4 heures. Les résultats figurent dans le Tableau 2.

### EXEMPLE 12:

On opère comme dans l'exemple 3 mais on utilise CoCl₂ à la place de Co(acac)₂. La réaction est arrêtée au bout de 3 heures et demie. Les résultats figurent dans le Tableau 2.

### EXEMPLE 13:

On opère comme dans l'exemple 2 mais on utilise comme ligand le diphéylphosphinobutane à la place du dppe. La réaction est arrêtée après 3h. Les résultats figurent dans le Tableau 2.

### EXEMPLE 14:

On opère comme dans l'exemple 2 mais on engage comme sel de cobalt le triacétylacetonate de cobalt à la place d'un cobalt bivalent. Après 7 heures on obtient les résultats qui figurent dans le Tableau 2.

### EXEMPLE 15:

On opère comme dans l'exemple 2 mais on utilise comme matière première un ester méthylique d'huile de tournesol non conjugué. Une partie du linoléate se conjugue, soit 16 % du C18:2. Une fraction reste sous la forme de C18:2 conj (10,2 %) et une autre fraction donne un produit d'addition (7,7 % par rapport au C18:2 original).

### EXEMPLE 16:

On opère comme dans l'exemple 2 mais on engage comme ligand la triphénylphosphine avec un rapport molaire ligand/cobalt = 1. Les résultats sont indiqués au Tableau 2.

### EXEMPLE 17 :

On opère comme dans l'exemple 16 mais avec un rapport molaire ligand/cobalt égal à 2. Les résultats sont indiqués en Tableau 2.

### EXEMPLE 18:

On opère comme dans l'exemple 2 mais on utilise le chlorure de cobalt sans solvant. Les résultats sont indiqués au Tableau 2.

### EXEMPLE 19 :

On opère comme dans l'exemple 12 mais on utilise comme ligand PCl₃ avec un rapport molaire Co/PCl₃ = 1:2. Après 3 heures on obtient le résultat indiqué au Tableau 2.

### EXEMPLE 20 :

On opère comme dans l'exemple 2 sauf que le ligand est le dicyclopentyl, 4-pyridyléthylidène-phosphane ("Cytec"), avec un rapport molaire 1:1 par rapport au cobalt.

### EXEMPLE 21 :

Dans cet exemple on utilise des conditions analogues à l'exemple 3 avec le dichloroéthane comme solvant et avec pour ligand la tributylphosphine introduite avec un rapport molaire 1:2 pour le rapport Co/Ligand. Après 3 heures le résultat obtenu est indiqué au Tableau 2.

### EXEMPLE 22 :

On utilise les mêmes conditions que dans l'exemple 2 sauf que le rapport molaire Co/réducteur = 1:5. Les résultats sont indiqués au Tableau 2.

### EXEMPLE 23 :

Les conditions sont identiques à l'exemple 3 sauf que le ligand est le triméthylphosphite. Les résultats sont indiqués au Tableau 2.

### EXEMPLE 24 :

Ici on utilise la bipyridyle avec un rapport molaire Co/ligand = 1:1. Les résultats sont indiqués au Tableau 2.

### EXEMPLE 25 :

Dans cet exemple on utilise la trioctylphosphine oxyde POBu3 avec un rapport molaire cobalt/ligand de 1: 2.

### EXEMPLE 26 :

On fait réagir Col₂ (2,5g) et le dppe (3,2g) dans 40 ml d'isopropanol et 15ml d'éther à 30 °C. On agite à 30 °C. Il se forme une solution bleue qui vire au vert. On ajoute 0,5 g de NaBH₄ en poudre sous azote. Après 30 minutes on constate la formation d'un précipité vert jaune que l'on filtre et que l'on lave avec de l'éther. Ce précipité est séché sous vide à 20 °C. Le produit formé ColH-dppe, est soluble dans le dlichloroéthane mais insoluble dans le toluène. Le complexe en présence de AlEt₃ introduit avec un rapport molaire de 10 et selon la procédure normale donne une faible conversion en produit ramifié de 17% en considérant la disparition de C18:2_{c} et de 11% en considérant la formation de produit ramifié.

### EXEMPLE 27 :

Le même complexe CoHl-dppe dans le dichloroéthane est introduit dans l'ester conjugué et l'on fait réagir le diéthylchloroaluminium utilisé dans un rapport molaire Co/réducteur de 1:10 à 80 °C pendant 3 heures ; il y a une conversion du C18:2_{c} de 52% et l'apparition de 36,6% de composé 1:1 et 1:2 ce qui correspond à 57 % du composé C18:2_{c} d'origine. Il n'y a pas de précipité noir mais à la fin une solution verte.

### EXEMPLE 28 :

On utilise le système décrit dans l'exemple 3 sauf que le rapport molaire entre le dppe et le cobalt est de 1,3 et le solvant le dichloroéthane. A 80 °C, après 3h, on obtient 65 % de conversion du C18:2_{c} et on constate le formation de 70 % de produit 1:1 et 1:2 par rapport au C18:2_{c} d'origine.

### EXEMPLE 29 :

On refait le même essai que dans l'exemple 28 mais cette fois ci avec un rapport molaire Co/réducteur de 1:4. Après 2 heures, on obtient une conversion de 30,6 % pour C18:2_{c} et 32,5 % de produit 1:1 et 1:2 par rapport à la théorie.

### EXEMPLE 30 :

On travaille cette fois-ci avec de l'ester méthylique de l'huile de tournesol qui a la composition suivante :
C16:0 = 6,75 %
C18:0 = 5,07 %
C18:1 = 19,8
C18:2 = 68,4
C18:2_{c} = 0
30 ml d'ester sont engagés avec 1,12 mmole de Co(aca)₂ et 0,28 mmole de Ni(acac)₂ en présence de 1,4 mmole de dppe et 14 mmole de DEAC (AlEt₂ Cl). A 100 °C et 3 MPa d'éthylène on obtient après 3 heures, 50,8 % de conversion du C18:2 avec la formation de 20,1 % de C18:2_{c} et 27 % de produit d'addition par rapport au C18:2.

### EXEMPLE 31 :

On prend 30 ml d'huile de tournesol dont la composition en acides gras est donnée dans le tableau suivant. On ajoute 1,12 mmole de Co(acac)₂, 0,28 mmole de Ni(acac)₂ et 1,82 mmole de dppe dans le toluène. On ajoute enfin à 100 °C 14 mmole de DEAC. Après 2,5 h à 3 MPa d'éthylène on obtient un composé que l'on transforme en ester méthylique pour pouvoir l'analyser en chromatographie en phase vapeur (CPV). Les résultats sont donnés dans le Tableau 1 ci-après :

**TABLEAU 1**

| | Produit de départ | Produit obtenu |
|---|---|---|
| C16:0 | 6,7 | 4,22 |
| C18:0 | 4,6 | 5,32 |
| C18:1 | 20,1 | 19,7 |
| C18:2 | 66,7 | 22,7 |
| C18:2_{c} | - | 21,9 |
| 1:1 | - | 16,7 |
| 1:2 | 0 | 5,76 |
| > C18 | 1,2 | - |
| > C22 | 0 | 2,55 |

Dans ce tableau,
- C18:2_{c} =: linoléate conjugué
- 1:1 =: produit d'addition avec 1 éthylène
- 1:2 =: produit d'addition avec 2 éthylène.

On constate que l'huile a été conjuguée et que l'éthylène a formé deux produits d'addition.

### EXEMPLE 32 :

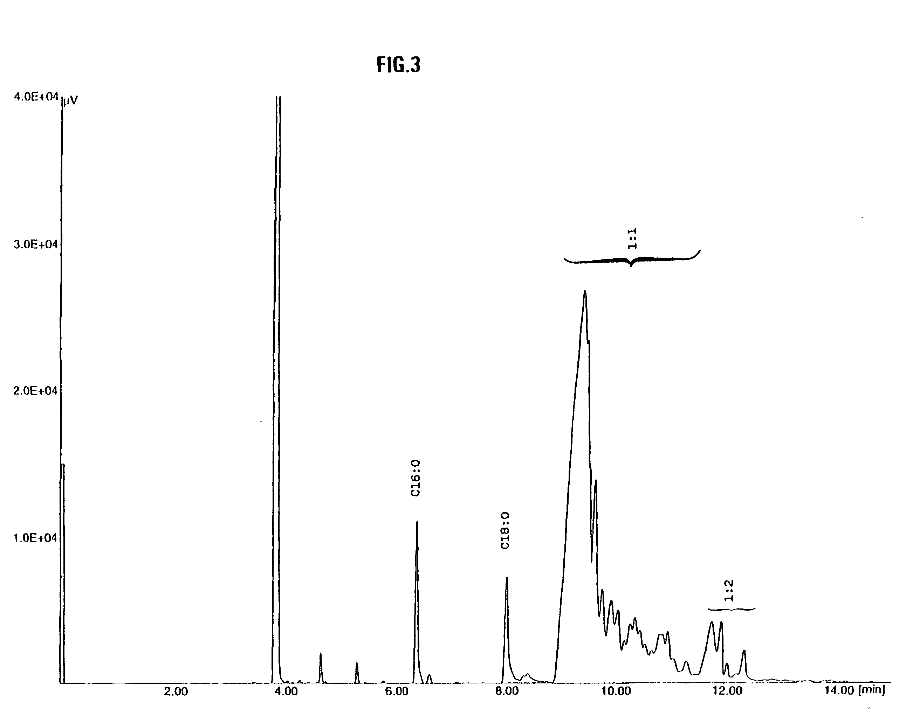

On hydrogène sans solvant avec le Pd/C (100 mg) le composé obtenu dans l'essai de l'exemple 12 après avoir lavé la solution de cet essai avec de l'eau et filtré le précipité formé. On cristallise après filtration du palladium sur charbon le composé dans l'acétone une fois à 0 °C et une seconde fois à -18 °C. Le composé d'addition après évaporation de l'acétone reste liquide à -18 °C. La chromatographie en phase vapeur indique que le composé contient 3 % de C16:0, 3% de C18:0 et 94% d'un produit d'addition principalement formé d'éthylstéarate de méthyle.
Le chromatogramme du produit est donné en annexe (Fig.3).
La RMN de l'hydrogène donne un signal de proton à 1,8 ppm correspondant à 1 proton sur un carbone tertiaire. C'est principalement la RMN du produit avant hydrogénation qui donne plusieurs indications sur le groupement vinylique entre 4,9 et 5 ppm absent dans le produit de départ et correspondant à une double liaison terminale soit un composé de type vinyloctadécénoate de méthyle.

Le Tableau 2 suivant regroupe les résultats des Exemples 1 à 25, qui concernent l'addition de l'éthylène sur un ester méthylique d'huile de tournesol conjugué. Dans ce tableau 2, on a considéré la conversion des linoléates conjugués et la conversion en produit d'addition par rapport à l'ester conjugué initial. Le catalyseur est un sel de cobalt réduit généralement par AlEt₂Cl dans le toluène, sauf lorsqu'il est indiqué un autre solvant ou un autre réducteur.

**TABLEAU 2**

| Addition de l'éthylène sur un ester méthylique d'huile de tournesol conjugué | | | |
|---|---|---|---|
| Exemple | Conversion des C18:2c | Conversion en Prod. d'addition | Remarques |
| 1 | 60 | 59,0 | ppt. noir |
| 2 | 62,4 | 62,4 | ppt. noir |
| 3 | 70 | 75,1 | léger ppt. noir (DCE) |
| 4 | 76,0 | 70,7 | ppt. noir |
| 5 | 56 | 46,6 | ppt. noir |
| 6 | 73 | 65,2 | ppt. rouge (TEA)(DCE) |
| 7 | 64,2 | 71,5 | pas de ppt |
| 8 | 31 | 21 | ppt rouge (TEA) |
| 9 | 40 | 45,5 | petit ppt. |
| 10 | 22 | 14,3 | ppt. noir |
| 11 | 33 | 18,7 | ppt. noir |
| 12 | 57 | 59 | pas de ppt (DCE) |
| 13 | 17 | 5,5 | petit ppt noir |
| 14 | 58 | 56,3 | ppt. noir |
| 15 | 29 ^{(*)} | 7,7 | ppt. noir |
| 16 | 21 | 13 | ppt. noir |
| 17 | 17 | 19,4 | ppt. noir |
| 18 | 70 | 64,7 | ppt. rouge (TEA) |
| 19 | 20 | 5,5 | pas de ppt., vert (DCE) |
| 20 | 11 | 5,5 | ppt. noir |
| 21 | 57 | 57 | petit ppt., vert, DCE |
| 22 | 55 | 53 | ppt |
| 23 | 15 | 4,3 | pas de ppt, DCE |
| 24 | 17 | 10,1 | ppt., DCE |
| 25 | 10 | 1 | ppt, DCE |

| | | | |
|---|---|---|---|
| (*) point de départ ester méthylique de l'huile de tournesol non conjugué, DCE = dichloroéthane, TEA = triéthylaluminium, ppt = précipité. | | | |

## Revendications

1. Procédé d'obtention d'un codimère **caractérisé en ce que** l'on additionne un composé monooléfinique sur un corps gras comportant au moins deux liaisons éthyléniques conjuguées ou non conjuguées, en présence d'un système catalytique comprenant au moins un composé du cobalt, au moins un composé réducteur et au moins un ligand.

2. Procédé selon la revendication 1 **caractérisé en ce que**, dans ledit système catalytique,
- le composé du cobalt est choisi parmi les sels inorganiques ou organiques du cobalt, les hydroxydes de cobalt, les organo-cobalt et les hydrures de cobalt ;
- le composé réducteur est un composé organoaluminique, un composé organomagnésien, un aluminoxane, le borohydrure de sodium, un hydrure de métal alcalin, éventuellement substitué par 1 à 3 groupements alcoxy ; et
- le ligand peut être choisi :
• parmi les composés du phosphore de formule PRₘX₃₋ₘ, avec m = 0, 1, 2 ou 3, R = aryle ou alkyle et X = halogène ; les phosphites P(OR)3, avec R = aryle ou alkyle ; les oxydes de phosphine POR₃ et les diphosphines de formule R₂P-(CH₂)ₙ-PR₂, avec R = aryle ou alkyle et n = 0 - 4.
• parmi les composés analogues de l'arsenic et de l'antimoine ;
• et parmi les ligands azotés, tels que les dérivés amidiques, les imines ou diimines et les dérivés pyridiniques.

3. Procédé selon la revendication 1 ou 2 **caractérisé en ce que** ledit composé monoléfinique est choisi parmi les hydrocarbures monooléfiniques comme l'éthylène, le propylène ou le butène -1.

4. Procédé selon l'une des revendications 1 à 3 **caractérisé en ce que** ledit corps gras comportant au moins deux liaisons éthyléniques est choisi parmi les corps gras diéniques ou polyéniques conjugués ou conjugables, le nombre d'atomes de carbone de la chaîne grasse comportant de 18 à 26 atomes de carbone sur la chaîne qui porte le groupe carboxylique, celui-ci étant lié à un alcool mono, di, tri ou tétrafonctionnel de 1 à 18 atomes carbone.

5. Procédé selon la revendication 4 **caractérisé en ce que** l'alcool est choisi parmi le méthanol, l'éthanol le néopentylglycol, le triméthylolpropane, le 2-éthylhexanol et le glycérol.

6. Procédé selon l'une des revendications de 1 à 5 **caractérisé en ce que** le système catalytique comporte, à titre de composé de cobalt, des halogénures, des acétylacétonates ou des carboxylates et, à titre de réducteur, un système à base d'alkylaluminium, substitué ou non, ou de l'aluminoxane ou des hydrures d'aluminium ou de bore, le rapport molaire entre le cobalt et le réducteur étant de 1 à 30, de préférence de 1 à 4.

7. Procédé selon l'une des revendications de 1 à 6 **caractérisé en ce que** le ligand introduit a pour formule PR₃ ou R₂P-(CH₂)ₙ-PR₂, avec n = 1,2,3 ou 4 et R = alkyle ou aryle, le rapport molaire entre le cobalt et le ligand étant de 1 à 10, de préférence de 1 à 3.

8. Procédé selon l'une des revendications de 1 à 7 **caractérisé en ce que** l'ester engagé est préalablement conjugué avec un système de type alcoolate alcalin ou conjugué au cours de l'addition avec le même système que celui qui permet l'addition d'oléfine.

9. Procédé selon la revendication 8 **caractérisé en ce que** l'ester engagé est conjugué au cours de l'addition d'oléfine ou en la précédant, avec un catalyseur au cobalt cocatalysé par des traces de métaux de transition de type fer, nickel, cuivre, rhodium ou palladium.

10. Procédé selon l'une des revendications 1 à 9 **caractérisé par le fait que** le catalyseur est un système mixte obtenu par réaction d'une part du complexe CoHXL₂ où X est un anion, de préférence un halogène, et L₂ une diphosphine, de préférence l'éthane bisdiphénylphosphine, ou une phosphine simple comme une alkylphosphine, introduite dans un rapport molaire 2:1 par rapport au cobalt, et d'autre part un réducteur tel que défini dans la revendication 2.

11. Procédé selon l'une des revendications 1 à 10, **caractérisé par le fait qu'**il comprend une étape ultérieure dans laquelle ledit codimère est hydrogéné en présence du catalyseur de codimérisation ou d'un catalyseur connu d'hydrogénation, éventuellement après filtration ou élimination du catalyseur de codimérisation.

12. Procédé selon la revendication 11 **caractérisé par le fait qu'**il comprend une étape ultérieure dans laquelle ledit codimère hydrogéné est purifié par élimination des composés saturés non ramifiés par cristallisation dans un solvant ou par distillation.

## Patentansprüche

1. Verfahren zum Erhalt eines Codimers, **dadurch gekennzeichnet, dass** eine monoolefinische Verbindung zu einem Fett, das wenigstens zwei konjugierte oder nicht-konjugierte ethylenische Bindungen aufweist in Gegenwart eines katalytischen Systems zugegeben wird, das wenigstens eine Verbindung von Cobalt, wenigstens eine reduzierende Verbindung und wenigstens einen Liganden umfasst.

2. Verfahren nach Anspruch 1, **dadurch gekennzeichnet, dass** in dem katalytischen System
- die Cobaltverbdinug aus den anorgnischen oder organischen Salzen von Cobalt, den Hydroxiden von Cobalt, Cobalt-organischen Verbidungen und den Cobalthydriden gewählt ist;
- die reduzierende Verbindung eine Aluminium-organische Verbindung, eine Magnesium-organische Verbindung, ein Aluminooxan, ein Natriumboranat, ein Alkalimetallhydrid gegebenenfalls durch 1 bis 3 Alkoxygruppen substituiert, ist; und
- der Ligand gewählt werden kann:
• aus den Phosphorverbindungen der Formel PRₘX₃₋ₘ, mit m= 0, 1, 2 oder 3, R= Aryl oder Alkyl und X= Halogen; den Phosphiten P(OR)₃ mit R= Aryl oder Alkyl; den Oxiden von Phosphin POR₃ und den Diphosphinen mit einer Formel R₂P-(CH₂)n-PR₂ mit R= Aryl oder Alkyl und n= 0 - 4.
• aus den analogen Verbindungen von Arsen und Antimon;
• und aus den stickstoffhaltigen Lignaden wie den amidischen Derivaten, den Iminen oder Diiminen und den pyridinischen Derivaten.

3. Verfahren nach einem der Ansprüche 1 oder 2, **dadurch gekennzeichnet, dass** die monoolefinische Verbindung aus den monoolefinischen Kohlenwasserstoffen wie Ethylen, Propylen oder 1-Buten gewählt ist.

4. Verfahren nach einem der Ansprüche 1 bis 3, **dadurch gekennzeichnet, dass** das Fett, das wenigstens zwei ethylenische Doppelbindungen umfasst, aus den konjugierten oder nicht-konjugierbaren dienischen oder polyenischen Fetten gewählt ist, wobei die Anzahl von Kohlenstoffatomen der Fettkette 18 bis 26 Kohlenstoffatome auf der Kette aufweist, die die carboxylische Gruppe trägt, welche mit einem mono-, di-, tri- oder tetrafunktionellen Alkohol mit 1 bis 18 Kohlenstoffatomen verbunden ist.

5. Verfahren nach Anspruch 1, **dadurch gekennzeichnet, dass** der Alkohol aus Methanol, Ethanol, Neopentlyglycol, Triethylolpropan, 2-Ethylhexanol und Glycerol gewählt ist.

6. Verfahren nach einem der Ansprüche 1 bis 5, **dadurch gekennzeichnet, dass** das katalytische System als Cobaltverbindung Halogenide, Acetylacetonate oder Carboxylate und als Reduktionsmittel ein System auf Basis von substituiertem oder nicht substituiertem Alkylaluminium oder Aluminiumoxan oder Hydriden von Aluminium oder Bor aufweist, wobei das molare Verhältnis zwischen Cobalt und dem Reduktionsmittel 1 bis 30, vorzugsweise 1 bis 4 ist.

7. Verfahren nach einem der Ansprüche 1 bis 6, **dadurch gekennzeichnet, dass** der eingeführte Ligand als Formel P R₃ oder R₂P-(CH₂)n-PR₂ mit n= 1, 2, 3 oder 4 und R= Alkyl oder Aryl hat, wobei das molare Verhältnis zwischen Cobalt und dem Ligand 1 bis 10, vorzugsweise 1 bis 3 ist.

8. Verfahren nach einem der Ansprüche 1 bis 7, **dadurch gekennzeichnet, dass** der eingesetzte Ester zuvor mit einem System vom Typ Alkalialkoholat oder bei der Zugabe mit dem System konjugiert ist, das die Olefinzugabe ermöglicht.

9. Verfahren nach Anspruch 1, **dadurch gekennzeichnet, dass** der eingesetzte Ester bei der Olefinzugabe oder vor ihr mit einem Cobaltkatalysator mit Cokatalyse durch Spuren von Übergangsmetallen vom Typ Eisen, Nickel, Kupfer, Cobalt, Rhodium oder Palladium konjugiert wird.

10. Verfahren nach einem der Ansprüche 1 bis 7, **gekennzeichnet, durch** die Tatsache, dass der Katalysator ein gemischtes System ist, das **durch** Reaktion des CoHXL₂-Komplexes, einerseits, wobei X ein Anion, vorzugsweise ein Halogen und L₂ ein Diphosphin, vorzugsweise Bisdiphenylphosphinethan oder ein einfaches Phosphin wie ein Alkylphosphin ist, das in einem 2:1 Molverhältnis im Verhältnis zum Cobalt eingeführt wird, und andererseits einem wie im Anspruch 2 definierten Reduktionsmittel.

11. Verfahren nach einem der Ansprüche 1 bis 10, **gekennzeichnet, durch** die Tatsache, dass es eine spätere Stufe umfasst, in der das Codimer in Gegenwart des Codimerisationskatalysators oder eines bekannten Hydrierungskatalysators gegebenenfalls nach Filtrierung oder Entfernung des Codimerisationskatalysators hydriert wird.

12. Verfahren nach Anspruch 11, **gekennzeichnet, durch** die Tatsache, dass es eine spätere Stufe umfasst, in der das hydrierte Codimer **durch** Entfernung der gesättigten, nicht-verzweigten Verbindungen **durch** Kristallisation in einem Lösungsmittel oder **durch** Destillation gereinigt wird.

## Claims

1. Process for obtaining a codimer, **characterized in that** a monoolefinic compound is added to a fatty substance that comprises at least two conjugated or unconjugated ethylene bonds, in the presence of a catalytic system that comprises at least one cobalt compound, at least one reducing agent, and at least one ligand.

2. A process according to claim 1 **characterized in that**, in said catalytic system :
- the cobalt compound is selected from the group consisting of the inorganic and organic cobalt salts, cobalt hydroxides organocobalt compounds and cobalt hydrides ;
- the reducing agent is an organoaluminum, an organomagnesium, an aluminoxane, sodium borohydride, an alkali metal hydride, optionally substituted by 1 to 3 alkoxy groups ;
- the ligand can be selected :
• from phosphorus compounds of formula PRₘX₃₋ₘ with m = 0, 1, 2 or 3; R = aryl or alkyl; X = halogen; phosphites P(OR)₃, with R = aryl or alkyl; phosphine oxides POR₃, and diphosphines of formula R₂P-(CH₂)ₙ-PR₂, with R = aryl or alkyl and n = 0-4;
• from the analogous compounds of arsenic and antimony;
• and from nitrogenous ligands, such as the amide derivatives, the imines or diimines, and the pyridine derivatives.

3. A process according to claim 1 or 2, **characterized in that** said monoolefinic compound is selected from among the monoolefinic hydrocarbons such as ethylene, propylene, or butene-1.

4. A process according to one of claims 1 to 3, **characterized in that** said fatty substance that comprises at least two ethylene bonds is selected from among the dienic or polyenic fatty substances that are conjugated or can be conjugated, whereby the number of carbon atoms of the fatty chain comprises 18 to 26 carbon atoms on the chain that carries the carboxylic group, with the latter being linked to a mono, di, tri or tetrafunctional alcohol with 1 to 18 carbon atoms.

5. A process according to claim 4, **characterized in that** the alcohol is selected from among methanol, ethanol, neopentylglycol, trimethylolpropane, 2-ethylhexanol, and glycerol.

6. A process according to one of claims 1 to 5, **characterized in that** the catalytic system comprises, as a cobalt compound, halides, acetylacetonates, or carboxylates and, as a reducing agent, a system with an alkylaluminum base, which may or may not be substituted, or aluminoxane or aluminum or boron hydrides, whereby the molar ratio between the cobalt and the reducing agent is 1 to 30, preferably 1 to 4.

7. A process according to one of claims 1 to 6, **characterized in that** the ligand that is introduced has as its formula PR₃ or R₂P-(CH₂)ₙ-PR₂, with n = 1, 2, 3 or 4 and R = alkyl or aryl, whereby the molar ratio between the cobalt and the ligand is 1 to 10, preferably 1 to 3.

8. A process according to one of claims 1 to 7, **characterized in that** the ester that is employed is previously conjugated with a system of the alkaline alcoholate type or conjugated during the addition with the same system as the one which allows the addition of olefin.

9. A process according to claim 8, **characterized in that** the ester that is employed is conjugated before or during the addition of olefin, with a cobalt catalyst that is cocatalyzed by traces of transition metals such as iron, nickel, copper, rhodium, or palladium.

10. A process according to one of claims 1 to 9, **characterized in that** the catalyst is a mixed system that is obtained by reaction, on the one hand, of the complex CoHXL₂, where X is an anion, preferably a halogen, and L₂ is a diphosphine, preferably ethane bisdiphenylphosphine, or a simple phosphine such as an alkylphosphine, introduced in a molar ratio of 2:1 relative to cobalt, and, on the other hand, a reducing agent as defined in claim 1.

11. A process according to one of claims 1 to 10 **characterized in that** it comprises a further step wherein the codimer is hydrogenated in the presence of the codimerization catalyst or a known hydrogenation catalyst, optionally after filtration or elimination of the codimerization catalyst.

12. A process according to claim 11, **characterized in that** it comprises a further step wherein the hydrogenated codimer is purified by elimination of the unbranched saturated compounds by crystallization in a solvent.
